# EUROPEAN PATENT APPLICATION

(11) **EP 2 169 435 A2**
(43) Date of publication of application: **31.03.2010**
(21) Application number: 09171028.5
(22) Date of filing: 22.09.2009
(51) Int. Cl.: G02B 6/00, G02B 6/28, G02B 6/032, A61B 18/22

(54) **Waveguides with aiming mechanisms**

(30) Priority: 26.09.2008 US 100480 P
(71) Applicant: Lumenis Ltd., 20692 Yokneam (IL)
(72) Inventor: Lewinsky, Reuven M., Alonei Aba 36005 (IL); Khen, Roee, Haifa 34990 (IL); Braitbart, Shlomi, Haifa 34990 (IL)
(74) Representative: Bevan, Emma

(57) **Abstract**

Disclosed are radiation systems and methods, including a system that includes a waveguide to direct radiation from a first radiation source, a covering to cover at least part of the waveguide and one or more optical fibers embedded in the covering to direct radiation from a second radiation source. Also disclosed is system that includes a hollow waveguide assembly including a proximal portion and a distal portion that can be coupled to the proximal portion at a coupling area, the hollow waveguide assembly being configured to direct radiation from a first radiation source to an output port at a distal end of the distal portion of the hollow waveguide assembly, and a coupling unit to couple into the distal portion of the hollow waveguide assembly radiation from a second radiation source and the radiation from the first source delivered through the proximal portion.

## Description

### CROSS-REFERENCE TO RELATED APPLICATIONS

This application claims priority to provisional U.S. application Serial No. 61/100,480, entitled "Waveguide With Embedded Aiming Mechanism," filed September 26, 2008, the content of which is hereby incorporated by reference in its entirety.

### BACKGROUND

The present disclosure is directed to radiation transmission via waveguides, and more particularly to waveguide-based radiation transmission systems and methods that include aiming mechanisms.

Laser systems are commonly used in many applications, including medical applications. For example, the CO2 laser (also written as "CO₂ laser"), commonly emitting radiation having a wavelength of approximately 10.6µm, is an effective surgical tool, especially for precise cutting of soft tissues. An Er:YAG laser system, emitting radiation with wavelength of approximately 2.94µm, is also considered to be an effective surgery tool. In some applications, the generated laser radiation is transmitted to a target area, such as human or animal tissue, as a free beam, either directly or through a scanning mechanism. In such applications, a direct line of sight generally exists between the laser source and the target tissue. The CO2 laser beam, for example, may be transferred through delivery devices such as articulated arms, scanners and others.

Frequently, however, targets that are to be irradiated with, for example, laser radiation, are not in direct line of sight with the laser source. Thus, a flexible delivery device such as a fiber or waveguide is required. For example, hollow glass waveguides with an internal silver coating is one example of a CO2 waveguide type delivery device which transfers the CO2 wavelength efficiently. However, attenuation of radiation in the visible range (e.g., having wavelengths of 05.-0.7µm) passing through such a waveguide is significant.

When using laser as a surgical tool, or for some other application (e.g., industrial applications), a basic safety feature is to enable the operator to have better control over the direction of the laser beam without damaging adjacent area. One such feature is the use of an aiming beam, usually created by a low power HeNe laser, Diode laser, LED or any other light source in the visible range that is used to indicate to the operator the spot or area where the non-visible laser radiation, e.g., CO2 laser radiation, is going to hit the tissue.

One challenge associated with the use of hollow waveguides (and/or similar radiation conduits) is that although aiming beams can generally be easily transmitted through solid silica fibers, the power/energy of an aiming beam can significantly be attenuated when it passes through a hollow waveguide because the principle of total internal reflection (which facilitates the low-loss transmission of aiming beams through solid optical fibers) does not apply when the aiming beam is passed through a hollow waveguide. The degree of power loss is generally proportional to the distance and curvature the beam encounters as it travels through the hollow waveguide. Practically, this means that an operator (e.g., a surgeon) may encounter problems when attempting to identify the target tissue at which he wishes to irradiate radiation (e.g., laser radiation). If the distance the aiming beam had to pass through a hollow waveguide were relatively short, it would be possible for an operator to still be able to view the aiming beam as it was being applied to the target area.

Another challenge associated with the use of waveguides, including hollow waveguides, is the typical radiation power/energy loss of about 5 to 10% per meter that generally translates into heat generation inside the waveguide, thus requiring use of a cooling mechanism. Another challenge is related to the relatively high cost of hollow waveguides that stems from the relatively complex manufacturing process employed to manufacture them. When such waveguides are employed in single-use application (e.g., when the waveguides have to be discarded after being used in the treatment of a patient), this cost could be significant. The cost consideration in using the above-described waveguides calls for solutions that will balance the efficacy of using such waveguides versus the cost factor associated with using them.

### SUMMARY

Disclosed herein are apparatus, devices, systems and methods to enable efficient and cost-effective transmission of an aiming beam to a treatment target, including when hollow waveguides are employed to deliver radiation energy. One such aiming mechanism is implemented using optical fibers to transmit visible radiation, with the optical fibers being embedded in a covering (jacket) that covers the waveguide (e.g., a hollow waveguide and/or other type of waveguides, including solid fibers). Such an arrangement avoids the need to pass the aiming radiation through the waveguide.

Also disclosed herein is a coupling device that enables splitting/dividing a hollow waveguide into separate parts (e.g., usually two, but more are possible) where one part, e.g., the distal part, of the waveguide may be disposable whereas another part, e.g., the proximal part, as well as the coupling device are non-disposable. The coupler, apart from connecting the two parts, is also configured to couple an aiming beam source into either the hollow central part of the hollow waveguide, into a jacket where solid silica fiber are embedded that may be used to transmit the aiming beam, or into the silica part of the waveguide where the aiming beam may pass more efficiently.

This division of the waveguide into parts has some benefits that help overcome some of the problems encountered when hollow waveguides are used as a single piece. One of these is the delivery of aiming beam in the waveguide. Because the power decrease of the aiming beam in the visible range traveling in a hollow waveguide is roughly exponential, with high sensitivity to bending of the waveguide, any shortening of the waveguide will reduce the power loss. Having the aiming beam source passing through only a part of the waveguide length thus enables utilizing a lower power aiming beam source.

Dividing the waveguide into two parts also has a cost advantage resulting from the fact that the proximal part is being used for at least several procedures (e.g., surgical procedures) whereas only the short distal portion of the waveguide (e.g., a single-use sterile product embedded in some surgical instrument) needs to be discarded, thus reducing the overall cost of the procedure.

In one aspect, a radiation system is disclosed. The radiation system includes a waveguide to direct radiation from a first radiation source, a covering to cover at least part of the waveguide and one or more optical fibers embedded in the covering to direct radiation from a second radiation source.

Embodiments of the radiation system include one or more of the following features.

The waveguide may include a hollow waveguide to direct radiation emitted by a CO2 laser source.

The one or more optical fibers may be embedded in the covering such that radiation emitted by the one or more optical fibers defines one or more of, for example, a ring and/or a spot (or some other shape) on a target area onto which the radiation from the first radiation source directed by the waveguide is applied.

The radiation system may further include the first radiation source, which may include a CO2 laser device, and the second radiation source, which may include a source to generate visible radiation.

The waveguide may include one or more of, for example, glass fibers, crystalline fibers, Sapphire fibers, Germanate glass fibers, Germanate glass fibers with Sapphire tips and/or hollow core fibers.

The first radiation source may include one or more of, for example, an Er:YAG laser device, a Ho:YAG laser device, an Nd:YAG laser device and/or at least one laser diode.

In another aspect, a method to perform radiation operations is disclosed. The method includes directing visible radiation from a second radiation source through one or more optical fibers embedded in a covering surrounding at least part of a waveguide configured to direct radiation from a first radiation source, and applying the visible radiation directed by the one or more optical fibers onto a target area that is to receive the radiation from the first radiation source.

Embodiments of the method may include any of the above-described features of the system, as well as one or more of the following features.

The method may further include directing the radiation from the first radiation source through the waveguide.

Directing radiation from the first radiation source through the waveguide may include directing radiation from a CO2 laser source through a hollow waveguide.

Applying the visible radiation directed by the one or more optical fibers may include applying the visible radiation onto the target area such that the applied visible radiation defines one or more of, for example, a visible ring-shape and/or a spot on the target area.

The target area may be, for example, a tissue and/or an organ of a patient.

The method may further include coupling the radiation generated by the first radiation source into the hollow waveguide.

The method may further include coupling the radiation generated by the second source into the one or more optical fibers.

In a further aspect, a method to manufacture a laser system to generate an aiming beam defining a section of a target area onto which radiation from a first radiation source is to be applied is disclosed. The method includes performing an extrusion process to form a covering that is to be fitted on a waveguide to direct radiation from the first radiation source, and embedding one or more optical fibers in the covering, the one or more optical fibers configured to direct radiation from a second radiation source to generate visible radiation.

Embodiments of the method may include any of the above-described features of the system and the first method, as well as one or more of the following features.

The method may further include fitting the covering over the waveguide.

Fitting the waveguide may include fitting the covering over a hollow waveguide configured to transmit radiation from a CO2 laser source.

In yet another aspect, a radiation system is disclosed. The system includes a hollow waveguide assembly including a proximal portion and a distal portion that can be coupled to the proximal portion at a coupling area, the hollow waveguide assembly configured to direct radiation from a first radiation source to an output port at a distal end of the distal portion of the hollow waveguide assembly, and a coupling unit to couple into the distal portion of the hollow waveguide assembly radiation from a second radiation source and the radiation from the first source delivered through the proximal portion.

Embodiments of the radiation system may include any of the above-described features of the first system and the first and second methods, as well as one or more of the following features.

The system may further include the first radiation source, which may includes one of, for example, a CO2 laser device and/or an Er:YAG laser device. The system may further include the second radiation source, which may include a source to generate visible radiation. Alternatively another wavelength may be coupled, e.g. Nd:YAG, which can be used for coagulation of blood vessels.

The proximal portion may be spatially separated from the distal portion such that in at least part of the coupling area the radiation from the first source and the radiation from the second source travel outside the proximal portion and the distal portion of the hollow waveguide assembly.

The coupling unit may include a beam combiner positioned approximately at the coupling area. The beam combiner may be configured to direct the radiation from the first radiation source and the radiation from the second radiation source towards the distal portion of the hollow waveguide assembly.

The beam combiner may be substantially transparent in one direction for a wavelength of one of the radiation from the first radiation source and the radiation from the second radiation source, and may be substantially reflective in another direction to another wavelength of the other of the one of the radiation from the first radiation source and the radiation from the second radiation source (e.g., the beam combiner may be transparent for one wavelength and reflective for another wavelength).

The coupling unit may further include an optical focusing element to focus the combined radiation from the first radiation source and the radiation from the second radiation source towards the distal portion of the hollow waveguide assembly.

The system may further include a housing to retain one or more of, for example, the coupling unit and/or the second radiation source.

The system may further include a purge gas mechanism to perform one or more of, for example, cool the hollow waveguide assembly, clean the hollow waveguide assembly and/or clean the housing and keep away smoke and tissue debris from the distal end of the waveguide.

The purge gas mechanism may include a gas entrance port defined in a first location on an exterior of the housing to enable coupling of a first purge gas into the coupling unit to be directed to the distal portion of the hollow waveguide assembly, and a gas exit port defined in a second location on the exterior of the housing to enable a second gas received in the coupling unit through the proximal portion of the hollow waveguide assembly to be removed from the coupling unit.

The purge gas mechanism may further include an isolation wall to define a first chamber and a second chamber inside the housing, the first chamber being decoupled from the second chamber such that the first gas delivered into the first chamber is substantially prevented from entering the second chamber, and such that the second gas delivered into the second chamber is substantially prevented from entering the first chamber. Thus, the purge gas being passed through the proximal portion of the waveguide may be substantially different from the purge gas being passed through the distal portion of the waveguide. For example, when used for laparoscopic surgery, the gas passing through the proximal portion of the waveguide may be air which is readily available and does not need to be purified, while the gas being passed through the distal portion of the waveguide is CO2, which is generally used for such surgery.

The distal portion of the hollow waveguide assembly may include a disposable hollow waveguide coupleable to the coupling unit through a coupler.

In a further aspect, a method to perform radiation operations is disclosed. The method includes coupling visible radiation from a second radiation source into a distal portion of a hollow waveguide assembly, the hollow waveguide assembly including a proximal portion, coupleable to a first radiation source (e.g., for tissue treatment purposes), and the distal portion being coupled to the proximal portion at a coupling area. The method also includes applying the visible radiation through an output port located at a distal end of the distal portion of the hollow waveguide assembly onto a target area that is to receive the radiation from the first radiation source.

Embodiments of the method may include any of the above-described features of the first and second systems and the first and second methods, as well as one or more of the following features.

The method may further include directing radiation from the first radiation source to the output port located at the distal end of the distal portion of the hollow waveguide assembly, the radiation from the first radiation source passing through the hollow waveguide assembly.

Directing radiation from the first radiation source may include directing radiation generated by one or more of, for example, a CO2 laser device and/or an Er:YAG laser device.

The proximal portion may be spatially separated from the distal portion such that in at least part of the coupling area the radiation from the first source and the radiation from the second source travel outside the proximal portion and the distal portion of the hollow waveguide assembly.

Coupling the visible radiation may include combining the radiation from the first radiation source and the radiation from the second radiation source to direct the combined radiation towards the distal portion of the hollow waveguide assembly.

Combining the radiation from the first radiation source and the radiation from the second radiation source may include directing the radiation from the first radiation source to a beam combiner that is substantially transparent to one of the radiation from the first radiation source and the radiation from the second radiation source and directing the radiation from the second radiation source to the beam combiner.

The method may further include focusing the combined radiation from the first radiation source and from the second radiation source towards the distal portion of the hollow waveguide assembly.

The method may further include directing gas to perform one or more of, for example, cooling the hollow waveguide assembly and/or cleaning the hollow waveguide assembly.

Directing the gas may include directing a first gas from the coupling area into the distal portion of the hollow waveguide assembly and directing a second gas through the proximal portion of the hollow waveguide assembly to the coupling area.

In another aspect, a coupling unit to couple visible radiation into a hollow waveguide assembly is disclosed. The coupling unit may include a housing having an entrance port to couple a proximal portion of the hollow waveguide assembly to the housing and an exit port to couple a distal portion of the hollow waveguide assembly to the housing and a beam combiner to combine radiation directed into the housing from a first radiation source and visible radiation generated by a second radiation source.

Embodiments of the coupling unit may include any of the above-described features of the first and second systems and the first, second and third methods, as well as one or more of the following features.

The coupling unit may further include a focusing element to focus the combined radiation from the first radiation source and the second radiation source towards the distal portion of the hollow waveguide assembly.

Details of one or more implementations are set forth in the accompanying drawings and in the description below. Further features, aspects, and advantages will become apparent from the description, the drawings, and the claims.

### BRIEF DESCRIPTION OF THE DRAWINGS

FIG. 1A is a schematic diagram of a laser system that includes an aiming mechanism.
FIG. 1B is a schematic diagram of a cross-section of an arrangement of a waveguide covered by a covering.
FIG. 2 includes schematic diagrams of a connector to couple light into optical fibers.
FIG. 3 is a schematic diagram of another implementation to couple optical radiation to an aiming mechanism of a laser system.
FIG. 4 is a schematic of a section of a laser system that includes a hollow waveguide and an aiming mechanism.
FIG. 5 is a flowchart of a procedure to perform laser operations.
FIG. 6 is a schematic diagram of a radiation system that includes a coupling unit to couple visible radiation into a hollow waveguide assembly.
FIG. 7 is a flowchart of a procedure to perform laser operations using, for example, the radiation system of FIG. 6.

### DETAILED DESCRIPTION

Disclosed herein are systems, devices and methods to deliver an aiming beam using an aiming mechanism that does not rely on the waveguide transmitting the principal (e.g., therapeutic) radiation component (e.g., the radiation component applied to perform intended operations on a target area) to deliver the aiming beam radiation. In some embodiments, the aiming mechanism includes optical fibers embedded in a covering (jacket) structure that surrounds the waveguide transmitting the principal radiation component(s).

Also disclosed herein are systems, devices and methods, including a system in which an aiming beam is coupled to a distal portion of a hollow waveguide configured to deliver radiation (e.g., having wavelengths in the infrared range of the electromagnetic spectrum). In some embodiments, a radiation system is provided that includes a hollow waveguide with a proximal portion and a distal portion that can be coupled to the proximal portion at a coupling area, and a coupling unit to couple into the distal portion of the hollow waveguide radiation from a second radiation source and the radiation from the first source delivered through the proximal portion.

### Optical Fibers Embedded in a Jacket Covering a Waveguide

As noted, in some embodiments, systems, devices and methods are described to generate aiming beams for use with surgical instruments, as well as with other types of applications (e.g., industrial applications). For example, a radiation system is provided that includes a waveguide to direct radiation from a first radiation source, such as for example, a CO2 laser device, and a covering (e.g., a jacket manufactured from some polymer) to cover at least part of the waveguide. One or more optical fibers are embedded in the covering to direct radiation from a second radiation source (e.g., laser diodes, LEDs, incoherent light sources, etc.) to the distal ends of the one or more fibers, whereupon radiation from the second radiation source is emitted on a target surface (e.g., tissue, organs) to define the target area onto which the radiation from the first source is to be applied. The radiation from the first source irradiating the defined target area interacts with the material of the surface (e.g., human tissue) to thus cause some therapeutic effect (e.g., tissue ablation).

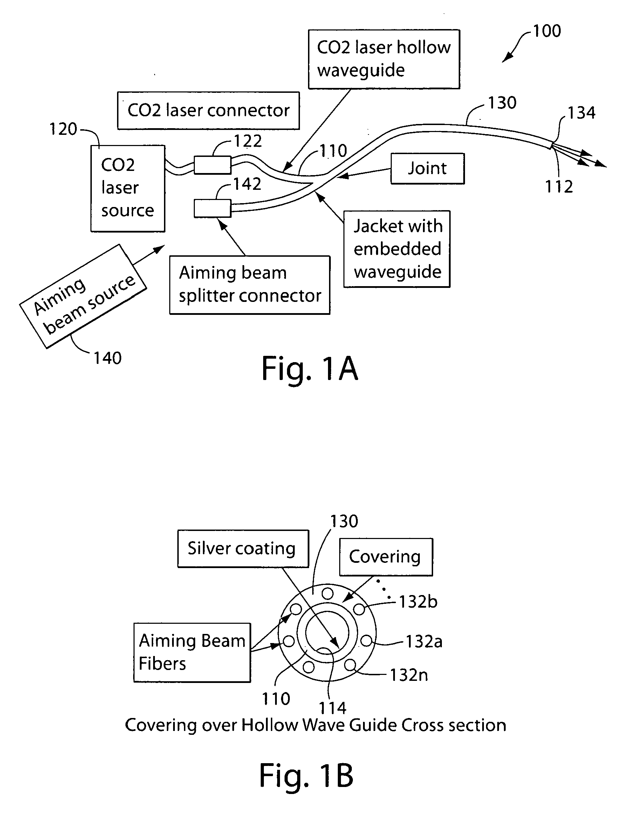

Referring to Figure 1A, a schematic diagram of a radiation system 100 that includes an aiming mechanism is shown. The radiation system 100 includes a waveguide 110 to direct radiation from a first radiation source 120, e.g., a CO2 laser generating radiation having a typical wavelength of approximately 10.6µm, that is coupled to one end of the waveguide 110. The radiation from the first radiation source is coupled using, for example, a connector 122 (e.g., a CO2 laser connector). Suitable laser connectors to connect the laser generating device to the waveguide (or conduit) may include, for example, laser SMA connectors, laser S-T connectors, etc. Other coupling arrangements (e.g., based on arrangements of optical elements) may also be used. The radiation coupled to the waveguide 110 is transmitted through the waveguide and emitted from a distal end 112 of the waveguide onto a target surface (e.g., human tissue). In some embodiments, the first radiation source to generate the radiation may include, for example, an Er:YAG laser system (that typically operates to generate radiation having a wavelength of approximately 2.94µm), a Ho:YAG laser system (used, for example, for urological applications) typically operating to generate a wavelength having a wavelength of approximately 2.1µm and/or Nd:YAG laser system emitting radiation having a wavelength of approximately 1.06µm. Other suitable laser devices may include, in some embodiments, at least one laser diode (which may be arranged in a diode array). The at least one laser diode may include a quantum-well laser based on Antimonide (Sb) compounds such as, for example, In(A1)GaAsSb-based compounds, GaSb-based compounds, etc. Suitable laser devices based on laser diodes include laser diode manufactured, for example, by Power Photonic Corporation of Stony Brook, New York. In some embodiments, the first radiation source may include a specially doped fiber laser such as, , for example, erbium-doped fluorozirconate fibre laser. Other types of radiation sources may also be used.

The type and/or configuration of the waveguide 110 to deliver the radiation generated by the first radiation source 120 may be based, at least in part, on the particular radiation source used. For example, in circumstances in which the first radiation source is a CO2 laser device, the waveguide 110 may be a hollow waveguide adapted to direct radiation generated by a CO2 laser device. Such a hollow waveguide may include a silica tube whose internal surface is coated with, for example, silver or other types of metals or waveguides made of polymeric layers. In some embodiments, the structure of a CO2 waveguide, such as the waveguide 110, may include several layers. The center of the waveguides may include the hollow part, defined by the surrounding layers, through which air or other gases may flow and inside which the IR radiation passes. Surrounding the hollow part is typically a thin film of Silver Iodine followed by another thin layer of silver metal. These layers may be surrounded by a silica layer with a typical wall thickness of several hundreds of microns, and the entire layered arrangement may be surrounded by a polymeric protective layer (sometime referred to as a buffer or coating.)

In some embodiments, for example, in implementations in which laser diode and/or laser systems to generate shorter wavelengths than those generated with a CO2 laser device, the waveguide may include one or more optical fibers adapted to transmit radiation (e.g., optical radiation) having such wavelengths of, for example, 1-10 µm. Suitable waveguides to transmit optical radiation having such wavelength includes, for example, glass or crystalline fibers, Sapphire fibers, Germanate glass fibers, a combination of Germanate glass fibers with Sapphire tip, hollow core fibers and/or any other suitable waveguide or radiation conduits to deliver laser energy. In some embodiments, the waveguide is composed of Germanate glass fiber and fused silica tips. In implementations where fiber-based waveguides are used, radiation couplers to couple radiation to these types of waveguides may be used.

As further shown in FIG. 1A, a covering 130 covers the waveguide 110 such that at least a portion of the waveguide 110 is disposed within the inner volume of the covering 130. In some embodiments, the covering 130 may function, among other things, as a protective shield (jacket) of the waveguide 110. Optical fibers are embedded within the covering 130. The covering 130 may be composed of a polymeric material, or some other flexible material, which may also act to increase the rigidity of the waveguide 110 to prevent it from accidental severe bending. The covering (jacket) manufacturing process may be based on extrusion techniques for both the covering and the embedded fibers. In some embodiments, the process may include first manufacturing the covering, then inserting the aiming beam optical fibers into the covering and subsequently inserting the waveguide into the covering. Any other order of manufacturing and/or types of manufacturing procedure may be implemented, including manufacturing procedures based on performing an extrusion procedure for the whole process (i.e., extruding materials to form a structure that includes optical fibers embedded in a covering surrounding a waveguide).

With reference to FIG. 1B, a schematic diagram of a cross-section of the waveguide 110 covered by the covering 130 is shown. In the depicted implementation, the waveguide 110 is a hollow waveguide with a circular cross-section and with a silver coating 114 lining the inner surface of the hollow waveguide 110. In some embodiments, the silver coated waveguide is configured to transmit laser radiation having a wavelength of approximately 10.6µm generated by a CO2 laser device. As further shown in FIG. 1B, one or more optical fibers 132a-n to deliver radiation in the visible range, or near the infrared range, are embedded into the covering. Fewer or additional optical fibers may be embedded into the covering. The one or more optical fibers may include a set of silica or other material fiber optics with good delivery characteristics to deliver radiation in the visible range. The one or more optical fibers generally extend from the coupling point (i.e., the point at which visible optical radiation used to form the aiming beam is coupled to the one or more optical fibers) along the remaining length of the covering 130 to the distal end 134 of the covering 130. Visible optical radiation coupled to the one or more optical fibers 132a-n is thus emitted at the distal end of the fibers, located at the distal end of the covering 130, and is projected on the surface to be treated. In embodiments in which the covering has a substantially circular cross-section (e.g., the covering 130 covers a substantially cylindrical waveguide), the one or more optical fibers 132a-n may be embedded along the circumference of covering such that the optical radiation emitted from the distal ends of the one or more optical fibers 132a-n may either form a ring-shaped image on the surface on which the optical radiation applied, or alternatively may form a substantially a round spot on the surface if the dispersion of the aiming beam exiting each optical fiber is large enough and a line of sight to the target tissue from sufficient distance is available. For example, in some embodiments, at least three (3) fibers, and optimally five to seven (5-7) fibers may be used to create a shape of a ring visible on the tissue that defines the area to be irradiated with the radiation generated from the first source (e.g., a CO2 laser device). In the embodiment depicted in FIG. 1B, the one or more optical fibers 132a-n are arranged at substantial equal distance intervals. However, the one or more optical fibers 132a-n may be embedded in the covering 130 based on different types of arrangements (e.g., arrangements that cause emitted light to define a crescent or some other shape). In some embodiments, the radiation emitted from the first radiation source (e.g., the therapeutic radiation) would be applied substantially at the center of the ring-shaped visible target generated by the aiming beam (depending, at least in part, on the angle the fiber is oriented with respect to the tissue, and to the tissue shape). An operator of the system 100 can thus observe the visible aiming shape formed on the surface which delineates the area onto which radiation transmitted through the waveguide 110 and emitted from the distal end 112 of the waveguide 110 is to be applied.

With reference again to FIG. 1A, optical radiation in the visible range, generated by a second radiation source 140, is coupled to the one or more optical fibers 132a-n, using an optical splitter/connector 142. The radiation source 140 may generate low power (e.g., 5 milliwatts) optical radiation having wavelength(s) in the visible range. For example, a suitable radiation source includes one or more laser diodes, such as HeNe laser, Diode laser, LED to generate optical radiation having a typical wavelength of 0.5 to 0.7µm. In some embodiments, the second source of radiation may be a light source generating incoherent light. Under those circumstances, an optical filter may be coupled to the light source to filter the incoherent light to enable optical radiation of specific wavelengths (e.g., substantially coherent light corresponding to a particular wavelength) to be coupled into optical fibers 132a-n.

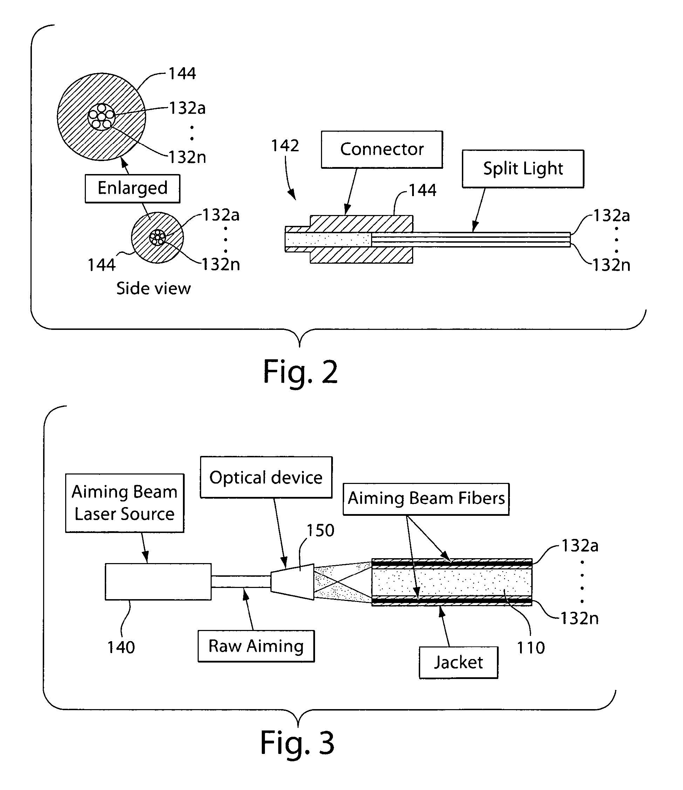

Referring to FIG. 2, schematic diagrams of an optical connector 142 to couple radiation from the second source to the one or more optical fibers is shown. In such embodiments, the aiming beam light enters a connector (e.g., a connector such as the SMA 905) using one or more optical devices (not shown) such as lenses. Received within a socket 144 of the connector 142 are the proximal end sections of the one or more fibers 132a-n. The end sections of the fibers 132a-n are received within the socket 144 such that they form a generally compact bundle where the optical fibers are placed next to the other fibers so that each of the one or more fibers small fiber gets a portion of radiation from the second radiation source directed into the connector 142. As shown in the cross-sectional diagram of the connector's socket and optical fibers arrangement, the level of radiation received by each of the bundled fibers will depend, at least in part, on the diameter of the fibers' ends (onto which the radiation coupled into the connector is applied). In circumstances where the fibers have substantially the same diameter size, each of the bundled fibers will receive substantially the same radiation level (provided that the entering beam power profile is relatively uniform and no significant deviation between the center and the periphery of entering beam exists). Setting this profile can be done using appropriate optical devices and design.

The bundled proximal end sections of the optical fibers are inserted into the covering surrounding the 110 waveguide, using, for example, a joint, or adapter, which places each of the optical fibers delivering an aiming beam into its place in the covering 130. The optical fibers 132a-n thus extend from their bundled location in the socket 144, through the covering 130 in which the optical fibers 132a-n are embedded, and culminating at the distal end of the covering 130 where the distal ends of the optical fibers 132a-n are located. As noted herein, the distal ends of the optical fibers 132a-n may be arranged along the circumference of the covering through which they pass such that, in some embodiments, the radiation emitted from the distal ends of the optical fibers 132a-n forms a ring of light points and/or a spot (or some other shape) on the surface on which the radiation generated by the first radiation source is to be applied.

In some embodiments, coupling the aiming beam radiation into the optical fibers 132a-n may be performed by coupling light into the fibers' ends that are already distributed along the circumference of the covering 130. Thus, with reference to FIG. 3, a schematic diagram of another implementation to couple light to an aiming mechanism of a laser system is shown. In the depicted implementation, visible aiming radiation generated by the second radiation source 140 is directed to one or more optical devices 150 such as, for example, a concaved divergent lens to cause radiation incident on the lens to diverge or disperse. The one or more optical devices 150 are configured (e.g., through selection of dimensions and lens' characteristics to control the optical behavior of the lens) to cause the incident radiation to be dispersed so that the aiming beam radiation is at least partly incident on the proximal ends of the optical fibers 132a-n such that at least some of the diverged radiation is coupled onto the optical fibers and is thus delivered via the optical fibers embedded in the covering 130 to the distal ends of the optical fibers whereupon the aiming beam radiation is emitted from those ends.

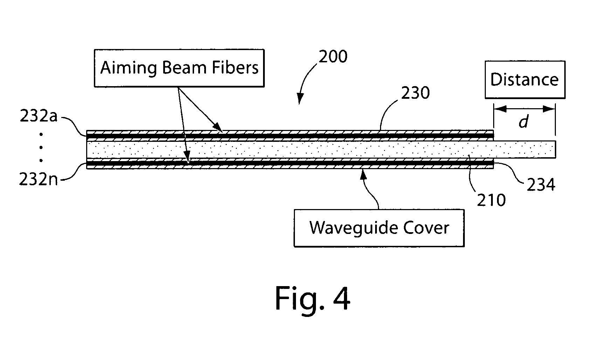

To protect the optical fibers embedded in the covering from possible damage resulting from tissue debris that may be released by application of the principal radiation (generated by the first radiation source), the covering, and thus the distal ends of the optical fibers, do not have to extend to cover the entire waveguide (i.e., so as to be flush with the edge of the distal end 112 of the waveguide). Referring to Figure 4, a schematic of a section of a laser system 200 that includes a waveguide is shown. The system 200 includes a covering 230, which may be similar to the covering 130 shown in FIGS. 1-3, embedded into which are one or more optical fibers 232a-n. Each of the optical fibers 232a-n may be similar in its structure, configuration and operation to the optical fibers described in relation to optical fibers 132a-n shown in FIGS. 1-3. The covering 230 surrounds a waveguide 210 which may be a hollow waveguide configured to transmit radiation generated by a CO2 laser device, or any of the other waveguides described in relations to FIGS. 1-3. In the embodiments of FIG. 4, the distal edge 234 of the covering 230 may extend to a position that is a distance *d* away from the edge of the distal end of the waveguide. In such implementations, stray debris (some of which may be moving at high speed) resulting from application of the radiation from the first radiation source is less likely to hit the distal ends of the optical fibers, thus extending the life of the optical fibers (and with it the life of the system). By having a sufficient number of embedded fibers in the jacket, the device reliability increases because even if one or even few of the fibers are blocked, the operator may still see the aiming beam.

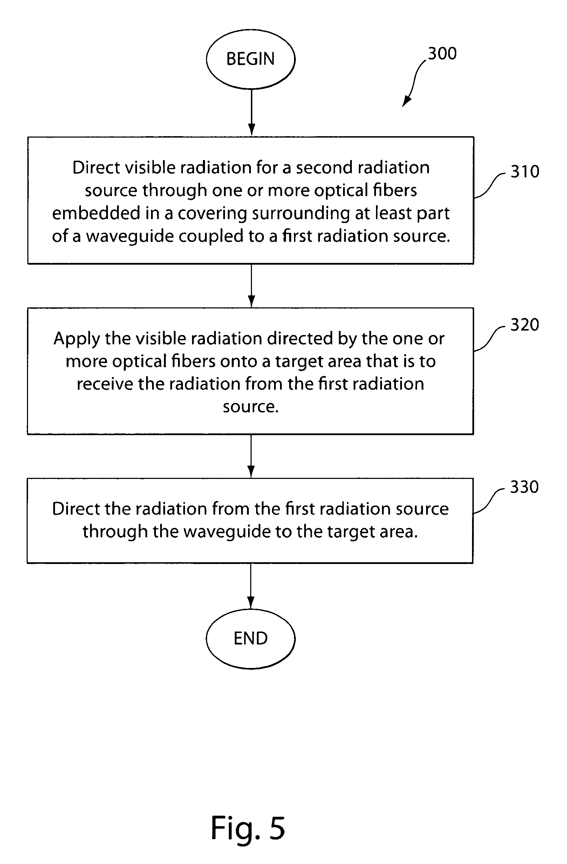

Referring to FIG. 5, a flowchart of a procedure 300 to perform radiation operations is shown. Visible radiation, generally low power radiation generated by a second (visible) radiation source, is directed 310 through one or more optical fibers embedded in a covering surrounding a waveguide coupled to a first radiation source that generates a principal radiation component (to perform an intended operation or procedure). In some embodiments, the waveguide may include a hollow waveguide such as the waveguide 110 shown in FIG. 1, configured to transmit radiation generated by a CO2 laser device, whose inner surface is coated with a metallic material such as silver. The visible radiation to form the aiming beam may be coupled to the optical fibers using a connector such as an SMA 905 connector (or any other suitable connector) or by using other types of optical devices (e.g., a divergent lens) to direct radiation incident on the optical device(s) to the ends of the optical fibers that are already arranged in the respective positions along the circumference or perimeter of the covering. The covering through which the optical fibers pass surrounds at least part of the waveguide. For example, in some embodiments, the covering extends to a distance d from the edge of the distal end of the waveguide (e.g., in implementations where some protection is required for the optical fibers from debris resulting from irradiation of the area treated with the radiation from the first radiation source).

The visible radiation delivered through the optical fibers embedded in the covering is applied 320 onto the target area that is to receive the radiation from the first radiation source (delivered through the waveguide). In some embodiments, the projected visible radiation defines a visible ring-shape image and/or a spot (and/or some other shape) on the surface to be irradiated. The aiming image defined by the visible radiation thus enables an operator to more accurately aim and apply the radiation from the first radiation source (which may be high power radiation that could potentially cause injury or damage to the treated area if not properly aimed)

Having identified, in some embodiments, the location to which the principal (e.g., therapeutic) radiation is to be applied, the laser radiation from the first radiation source is directed 330 through the waveguide to the identified location (the target area). In some embodiments, the radiation is generated by a CO2 laser system, and coupled (e.g., using a radiation connector) into the waveguide, e.g., a hollow waveguide whose inner surface is coated with a metallic material such as silver. In some embodiments, radiation from other types of radiation sources, such as laser diodes, Er:YAG laser system, Ho:YAG laser systems, Nd:YAG laser systems, etc., may be generated and directed through appropriate waveguides configured to transmit radiation generated by the laser system employed.

### Coupling Aiming Radiation into a Hollow Waveguide

As noted, in some implementations of the systems, apparatus and methods described herein, an aiming beam may be directed to the target area via the waveguide that is used to transmit the principal (treatment) radiation component. Thus, in some embodiments, a radiation system is provided that includes a hollow waveguide assembly comprising a proximal portion and a distal portion. In some embodiments, the proximal portion and the distal portion constitute separate hollow waveguide parts. In some embodiments, additional waveguide parts may be used. The distal portion is coupleable (i.e., can be coupled) to the proximal portion at a coupling area. The hollow waveguide assembly is configured to direct radiation from a first radiation source (e.g., the source generating treatment or therapeutic radiation) to an output port at a distal end of the distal portion of the hollow waveguide assembly. The radiation system also includes a coupling unit to couple into the distal portion of the hollow waveguide assembly radiation from a second radiation source and the radiation from the first source delivered through the proximal portion.

In some embodiments, the waveguide is split into two separate parts, and a coupling unit (e.g., an adaptor or some other adaptive device) is used to connect the two (or more) parts. The waveguide's parts can be similar to one another or different by their internal diameter, length, outer protective layer or any other attribute. Implementing the waveguide and radiation system as a system that includes separate waveguide parts enables the use of a reusable proximal part and a disposable distal part that are coupled to each other through the coupling unit. Thus, instead of discarding the entire waveguide (or any other type of radiation conduit), after completing the intended procedure (e.g., treatment on a patient) only the distal part of the waveguide may be discarded, and a replacement disposable distal part may be connected to the proximal part via the coupling unit when performing another procedure.

In some variations, the coupling unit (also referred to as a connection box or a junction box) connecting the waveguide parts is used to introduce another light of a different wavelength in the visible range to serve as an aiming beam or another treatment beam. Such aiming beam radiation will not have to pass the entire length of the waveguide (i.e., the sum of the lengths of the proximal and distal portions), but rather will only have to traverse the distal part and thus the aiming beam radiation will undergo less attenuation. The coupling unit (e.g., the connection box) is structured to receive at different ports respective ends of the proximal and distal portions of the waveguide. The radiation from the first source (e.g., the treatment or therapeutic radiation) and the radiation from the second source (e.g., the visible radiation constituting the aiming beam) may be combined in the coupling unit, and the combined radiation is coupled to the distal portion of the hollow waveguide assembly, whereupon the combined radiation, which includes a visible component and a therapeutic radiation component, are irradiated at the target area. In some embodiments, the radiation from the first source is coupled into the distal portion after the aiming beam has already been coupled into the distal portion so as to illuminate and identify the area onto which the radiation from the first source is to be applied.

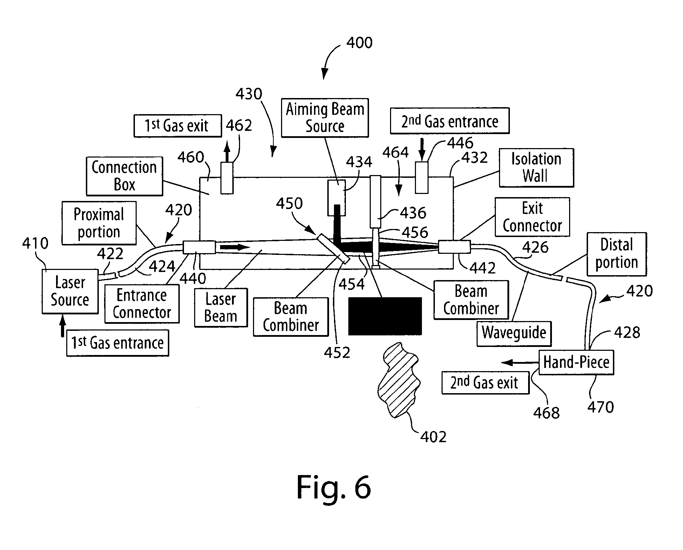

With reference to FIG. 6, a schematic diagram of a radiation system 400 that include a coupling unit 430 to couple visible radiation into a hollow waveguide assembly is shown. The system 400 includes a radiation source 410 that, in some implementations, generates radiation for treating tissue/organs having a wavelength in the infrared (IR) range. As noted herein, suitable radiation sources include, for example, a CO2 laser device, an Er:YAG laser device, etc. Laser devices to generate radiation having wavelengths in other ranges may also be used.

The radiation generated by the radiation source 410 is coupled to a proximal end 422 of a waveguide assembly 420 (or some other conduit to transmit radiation). The radiation source 410 may be coupled to the waveguide assembly via suitable laser connectors such as, for example, laser SMA connectors, laser S-T connectors, etc., and/or other coupling arrangements (e.g., based on arrangements of optical elements). In circumstances where the generated treatment radiation is in the IR range, waveguides/waveguide assemblies, configured to transmit IR radiation may include hollow waveguides, similar to the hollow waveguide 110 depicted in FIG. 1A, and may include a hollow waveguide having an internal surface coated with silver and/or other types of metal. In some implementations, the waveguide assembly 420 includes a proximal portion 424 and a distal portion 426 which, in some embodiments, may each be a separate waveguide (e.g., hollow waveguide). The proximal end 422 is located on the end of proximal portion 424 through which radiation from the radiation source 410 is coupled to the waveguide. As further shown in FIG. 6, in some embodiments, coupling of the distal portion 426 of the waveguide 420 to the proximal portion 424 is implemented using the coupling unit 430, also referred to as a connection box, that is positioned in an area referred to as the coupling area.

The coupling of the proximal portion 424 and the distal portion 426 does not necessarily require direct physical contact of the two portions. Rather, in some implementations, radiation from the source 410 traveling through the proximal portion is emitted through an end of the proximal portion 424 that is coupled into the coupling unit 430, whereupon the emitted radiation travels outside the hollow waveguide assembly 420 en route to the distal portion 426 of the waveguide assembly. Thus, in some embodiments, the proximal portion 424 of the waveguide assembly is spatially separated from the distal portion 426 such that in at least part of the coupling area, the radiation source 410 travels outside the proximal portion and the distal portion of the waveguide assembly. Furthermore, in some embodiments, the proximal portion 424 and the distal portion 426 are two separate portions that may each have different dimensions and physical attributes (e.g., different internal/external diameters, different materials, etc.)

The coupling unit 430 is configured to couple radiation constituting the aiming beam (e.g., optical radiation in the visible range) into the hollow waveguide assembly 420. In some embodiments, the coupling unit includes a housing 432 defining an interior through which visible radiation produced by a visible radiation source, such as the source 434 disposed inside the housing 432 can be combined with the treatment radiation received from the proximal portion 424 of the delivery system. Thus, the housing unit 430 may also include a radiation entrance connector 440 connectable to the proximal portion 424 (e.g., at a second end of the portion 424), and an exit connector 442, to connect the distal portion 426 of the hollow waveguide assembly 420 to the housing unit 430.

Similar to the radiation source 140 depicted in FIG. 1A, suitable radiation sources to generate the visible radiation that is to be coupled into the hollow waveguide assembly 420 (or, more specifically, into the distal portion 426 of the waveguide assembly 420) include low power Diode lasers, LED's or any other light source in the visible range that, when emitted from the emitting end of the waveguide and applied onto the target area, is used to indicate to the operator the area where the principal radiation component is going to be applied.

The coupling mechanism of the coupling unit 430 may include, in some embodiments, a beam combiner 450. The beam combiner 450 is configured to direct the radiation from the first radiation source 410 and the radiation from the second radiation source towards the distal portion 426 of the hollow waveguide assembly 420. In some embodiments, the beam combiner 450 is substantially transparent to one of the radiation components (e.g., from the first radiation source 410 or the second radiation source 434) such that at least some of the radiation from that radiation source passes through the beam combiner towards the distal portion of the waveguide. The beam combiner 450 may also be substantially reflective to the radiation from the other radiation source such that radiation from that source is reflected towards the distal portion of the hollow waveguide assembly.

In some implementations, the beam combiner 450 may include, for example, a plate 452 having a reflective surface 454, with the plate 452 being in a slanted orientation relative to the walls of the housing 432 of the coupling unit 430. For example, in some embodiments, the beam combiner may be implemented as a piece of glass onto which several coatings are applied.
These coatings may be transparent to some wavelengths but reflect other wavelengths. By applying the various layers to the piece of glass, the nature of the beam combiner can be controlled. Thus, in some implementations, the layer 454 layer may be transparent to IR but be reflective in the visible range, thus enabling combining the aiming beam with the main CO2 beam. Alternatively, in some embodiments, the beam combiner may be transparent to visible range radiation while being reflective to IR radiation. This may happen, for example, in situations where the treatment radiation is coupled to the coupling unit 430 in a direction and/or orientation that is similar to the direction and/or orientation in which the radiation from the source 434 hits the beam combiner in the implementation of FIG. 6.

In some implementations, the beam combiner may be implemented using a dichroic mirror that reflects, for example, visible light and transmits radiation generated, for example, by a CO2 laser device. When oriented in an angle of, for example, 40°-60°, at least some of the visible light radiation incident on the reflected surface 454 will be reflected towards the distal portion 426 of the hollow waveguide assembly 420.

In some embodiments, the plate 452 may be a rotateably adjustable plate so that the angular orientation of the plate 452 (and thus of the beam combiner) could be adjusted to control the angle of reflection of the radiation incident on the reflective surface 454. The coupling unit may thus also include actuating mechanisms (not shown) to control the angular orientation of the plate 452.

Other implementations of a beam combiner to combine the principal radiation and visible range radiation and direct the combined radiation towards the distal portion may also be used.

The beam combiner may combine several radiation components to direct them to a substantially common pre-determined optical path, or may direct individual radiation beams to the predetermined optical path without other radiation components being present (e.g., when only one radiation source is activated and is generating radiation).

As further shown in FIG. 6, the coupling unit also includes a focusing element 456, such as a lens (e.g., a biconvex lens) to converge the principal radiation and/or the aiming radiation directed by the beam combiner 452 of the coupling unit. The focused radiation is directed and coupled into the entrance of the distal portion 426 of the hollow waveguide assembly 420 that is in optical communication with the focusing element. In some implementations, multiple optical elements may be used to manipulate the radiation directed towards the distal portion of the waveguide (e.g., achieving the focusing operation required to direct and couple the radiation to the distal portion of the waveguide 420).

Thus, in operation, in some embodiments, visible optical radiation that is to be used to provide an aiming beam, is generated by a visible radiation source 434 (which may be disposed inside the housing of the coupling unit 430) and is directed towards the beam combiner 450, where upon the visible radiation is directed to the distal portion 426. Radiation treatment from the radiation source 410 is coupled into the proximal portion of the hollow waveguide assembly 420 and is emitted into the coupling unit 430. The emitted treatment radiation is directed to the beam combiner 450. In implementations in which the beam combiner 450 is configured to be transparent to the treatment radiation, the treatment radiation passes through the beam combiner. In some embodiments, radiation from additional sources (be it sources to generate radiation to perform procedures, including therapeutic procedures, or radiation to enable aiming or handling of the apparatus) may be generated and coupled into the coupling unit. The radiation components combined by the beam combiner are directed to a focusing element that focuses the combined radiation component to cause the focused radiation component to be coupled into the open end of the distal portion 426 of the waveguide 420 such that the radiation component are both guided by the hollow waveguide towards their destination (i.e., the target area 402 to be treated). Because the visible radiation is coupled into the hollow waveguide assembly 420 at a point that is much closer to the end of the waveguide than the entry point where the principal radiation is generated and coupled into the waveguide, the visible radiation coupled into the distal portion is not significantly degraded by the time it reached the distal end of the distal portion and emitted onto the target area 402. Thus, the power level of the visible radiation component emitted from a distal end 428 of the distal portion 426 will be high enough that an aiming beam applied to the target area 402 will be sufficiently visible to enable the operator (e.g., the surgeon) to see the aiming beam.

Generally, an operator (e.g., a surgeon) will initially activate the visible radiation source to generate visible radiation that is coupled to the distal portion and applied to the target area to identify the location onto which the principal radiation, once generated and directed via the waveguide, will be applied. Thus, after the operator has manipulated the distal portion of the waveguide (the distal portion may include a handle, or hand-piece, 470 that the operator can grasp, and/or other actuating elements) and identified the intended target location by moving the aiming beam emitted from a outlet port 468 so that the beam is applied to the desired location on the target area, the operator can then activate the first radiation source (or otherwise enable ongoing radiation to be directed to the coupling unit and/or the distal portion) so that the principal radiation is combined with the aiming radiation, and the combined radiation is then coupled into the distal portion of the waveguide and applied to the target area where the principal radiation component performs its intended procedure while the visible radiation component continues to be applied to the target area to indicate the location at which the treatment radiation is being applied. In some embodiments, once the treatment radiation is being applied to the target area, the aiming beam radiation may be suspended and the operator can then track the location on the target area to which the treatment radiation is being applied by following the resultant changes to the area (e.g., tissue) being operated upon (e.g., discoloration, carbonization, etc.)

In some implementations, the coupling unit includes a purge gas mechanism to, for example, cool the waveguide assembly and/or clean the waveguide assembly and the coupling unit from smoke and tissue debris that may have entered the waveguide's distal end and may therefore block the waveguide, damage it or simply degrade its performance (e.g., attenuate the radiation transmission). As both parts of the waveguide require purge gas, the coupling unit 430 has to enable gas (e.g., air or CO2) passed through the proximal part air to exit the coupling unit, and also enable optionally a second type of gas to be inserted into the distal portion 426 of the waveguide. Thus, as further shown in FIG. 6, in order to decouple these two gas flows, the coupling unit's internal volume is divided into two sealed chambers, namely a proximal chamber 460 through which the purge gas treating the proximal portion 424 of the waveguide is passed, and a distal chamber 464 into which purge gas is introduced and directed into the distal portion 426. To define the two chambers, an isolation wall 436, which, in some embodiments, may be positioned so that it extends from the focusing element 456, is used to divide the coupling unit 430 into the proximal and distal chambers which are substantially isolated from each other such that the respective gas flows in each chamber do not enter the other chamber. Thus, the purge gas being passed through the proximal portion of the waveguide may be substantially different from the purge gas being passed through the distal portion of the waveguide. For example, when used for laparoscopic surgery, the gas passing through the proximal portion of the waveguide may be air which is readily available and does not need to be purified, while the gas being passed through the distal portion of the waveguide may be CO2 which is generally used for such surgery.

In some embodiments, gas is inserted into distal chamber 464 via the gas entrance port 466, and is directed to the distal portion 426 of the waveguide 420. The gas used to clean the chamber 460 and the distal portion of the waveguide 420 should, in embodiments in which the radiation system 400 is used to treat patients, be one that does not harm a patient's tissue when it exits from the distal end 428 of the distal portion 426. Thus, a suitable gas that may be used to clean and cool the distal portion 426 and the distal chamber 464 may be CO2 gas, which is also routinely used to insufflate the patient's abdominal cavity during a laparoscopic procedure. As shown in FIG. 6, suitable gas, such as CO2 gas, that enters the distal chamber 466 via the entrance port 466 passes through the distal portion 426 of the waveguide assembly 420, and exits through an exit port 468 which may be situated, in some embodiments, near the hand-piece 470 used to manipulate the movement of the distal portion 426. The exit port 468 may also serve as the outlet through which insufflating gas is directed to a patient's tissue. As for the proximal chamber, because the gas used to cool and clean the proximal portion 424 of the waveguide 420 and the proximal chamber 460 does not come in contact with the patient's tissue, other types of gases may be used. For example, a regular air mixture may be passed through the proximal portion 424 and towards the proximal chamber 460. The gas used to purge the proximal portion of the waveguide and the proximal chamber is removed from the chamber 460 via a gas exit port 462. Optionally, the two chambers can be connected using a simple air tube so that only one gas source, e.g., CO2, will be used instead of having one supply for 464 and another at the entrance to the waveguide 422.

The coupling unit 430 enables separating the waveguide 420 into portions of different size and lengths. As noted, in some embodiments, the distal portion 426 of the waveguide does not have to be, for example, of the same dimensions (e.g., diameter) as the proximal portion. Additionally, as noted, because the proximal portion need not be in physical contact with the proximal portion (i.e., they may be spatially separated), when treating different patients, disposable distal portions may be used for different patients such that only the distal portion (which comes in direct contact with the patient being treated) needs to be regularly replaced, while a proximal portion connected to the treatment radiation source (e.g., the laser device) may be used repeatedly, so long that the proximal portion is substantially isolated from potential contaminants. Regular replacement of just the distal portion of the waveguide, instead of the entire waveguide, can thus result in significant cost savings.

To ensure that the proximal portion is substantially isolated from contamination, in some embodiments, the coupling unit 430 may be positioned and/or serve as the interface between the sterile and the non-sterile zones in an operating room (OR). The coupling unit therefore also includes an appropriate mechanism (e.g., anchoring mechanism) to secure the coupling unit to a desired place so that the coupling unit (and thus the entire radiation system 400) will be firmly supported, yet enable flexible maneuvering of the operating instrument (which may include the distal portion 426 of the waveguide 420).

In some embodiments, instead of having the aiming beam coupled into the hollow part of the distal portion 426, the structure of the distal portion may be made similar to the structure depicted in FIG. 1 so that the joint in FIG. 1A is placed inside the connection box 430. This implementation avoids the need to use a beam combiner, and is generally suitable for a "butt coupling" arrangement for transferring energy from the larger diameter portion 424 to the smaller diameter of the distal portion 426.

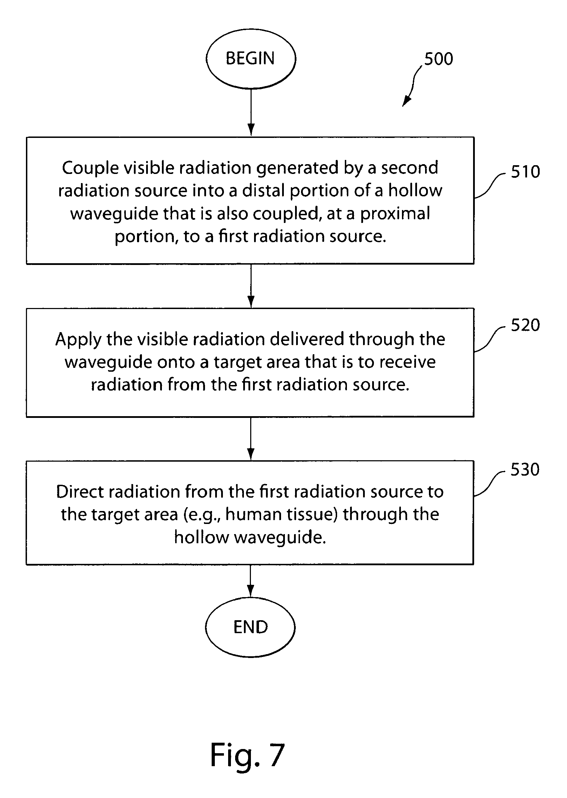

With reference to FIG. 7, flowchart of a procedure 500 to perform laser operations using, for example, the radiation system 400 depicted in FIG. 6, is shown. Visible radiation, generally low power radiation generated by a second radiation source, is coupled 510 into the distal portion of a hollow waveguide. In some embodiments, the hollow waveguide may be a hollow waveguide assembly whose inner surface is coated with a metallic material such as silver. The hollow waveguide assembly may include a proximal portion and the distal portion which is being coupled to the proximal portion at a coupling area. A first radiation source to generate the principal (treatment) radiation is coupled to the proximal portion of the waveguide. In some embodiments, the proximal and distal portions are spatially separated from each other such that in at least part of the coupling area, the treatment radiation from the first source travels outside either the proximal or distal portions of the waveguide. The visible radiation to form the aiming beam may be coupled into the distal portion of the waveguide using a coupling unit. In some embodiments, coupling the visible radiation into the distal portion of the waveguide includes using a beam combiner, such as the beam combiner 450 shown in FIG. 6, to combine the radiation from the first radiation source and the visible radiation from the second radiation source to direct the combined radiation towards the distal portion of the hollow waveguide assembly. The combined radiation may then be focused towards the distal portion of the hollow waveguide assembly using, for example, the focusing element 456 of the coupling unit 430 shown in FIG. 6.

The visible radiation delivered through the waveguide is applied 520 onto the target area (e.g., the target area 402) that is to receive the radiation from the first radiation source. The aiming image defined by the visible radiation applied onto the target area thus enables an operator to more accurately aim and apply the treatment radiation from the first radiation source.

Once the operator is satisfied that the aiming beam is pointing to the correct target, the operator can activated the first radiation source to cause generated radiation from the first source to be directed 530 through the proximal portion and enter the coupling unit, and thereafter be combined, in some embodiments, with the visible radiation so that the principal radiation component is coupled onto the distal portion of the waveguide and delivered to the target area to perform the intended procedure. Radiation from the first radiation source to be applied to the target area may be generated by a CO2 laser system. In some embodiments, radiation from other types of radiation sources, such as laser diodes, Er:YAG laser system, Ho:YAG laser systems, Nd:YAG laser systems, etc., may be generated and directed through appropriate waveguides configured to transmit radiation generated by the laser system employed.

Various embodiments of the subject matter described herein may be realized in digital electronic circuitry, integrated circuitry, specially designed ASICs (application specific integrated circuits), computer hardware, firmware, software, and/or combinations thereof. These various embodiments may include embodiment in one or more computer programs that are executable and/or interpretable on a programmable system including at least one programmable processor, which may be special or general purpose, coupled to receive data and instructions from, and to transmit data and instructions to, a storage system, at least one input device, and at least one output device. Some embodiments include specific "modules" which may be implemented as digital electronic circuitry, integrated circuitry, specially designed ASICs (application specific integrated circuits), computer hardware, firmware, software, and/or combinations thereof.

A number of embodiments of the invention have been described. Nevertheless, it will be understood that various modifications may be made without departing from the spirit and scope of the invention. Accordingly, other embodiments are within the scope of the following claims.

## Claims

1. A radiation system comprising:
a waveguide to direct radiation from a first radiation source;
a covering to cover at least part of the waveguide; and
one or more optical fibers embedded in the covering to direct radiation from a second radiation source.

2. The radiation system of claim 1, wherein the waveguide comprises a hollow waveguide to direct radiation emitted by a CO2 laser source.

3. The radiation system of claim 1, wherein the one or more optical fibers are embedded in the covering such that radiation emitted by the one or more optical fibers defines one of a ring and or a spot on a target area onto which the radiation from the first radiation source directed by the waveguide is applied.

4. The radiation system of claim 1 further comprising:
the first radiation source, the first radiation source comprising a CO2 laser device; and
the second radiation source, the second radiation source comprising a source to generate visible radiation.

5. The radiation system of claim 1 wherein the waveguide includes one or more of:
glass fibers, crystalline fibers, Sapphire fibers, Germanate glass fibers, Germanate glass fibers with Sapphire tips and hollow core fibers.

6. The radiation system of claim 1 wherein the first radiation source comprises one or more of: an Er:YAG laser device, a Ho:YAG laser device, an Nd:YAG laser device and at least one laser diode.

7. A radiation system comprising:
a hollow waveguide assembly including a proximal portion and a distal portion coupleable to the proximal portion at a coupling area, the hollow waveguide assembly configured to direct radiation from a first radiation source to an output port at a distal end of the distal portion of the hollow waveguide assembly; and
a coupling unit to couple into the distal portion of the hollow waveguide assembly radiation from a second radiation source and the radiation from the first source delivered through the proximal portion.

8. The radiation system of claim 7 further comprising:
the first radiation source, the first radiation source comprising one of: a CO2 laser device and an Er:YAG laser device; and
the second radiation source, the second radiation source comprising a source to generate visible radiation.

9. The radiation system of claim 7, wherein the proximal portion is spatially separated from the distal portion such that in at least part of the coupling area the radiation from the first source and the radiation from the second source travel outside the proximal portion and the distal portion of the hollow waveguide.

10. The radiation system of claim 7, wherein the coupling unit comprises:
a beam combiner positioned approximately at the coupling area, the beam combiner configured to direct the radiation from the first radiation source and the radiation from the second radiation source towards the distal portion of the hollow waveguide assembly; and
the coupling unit preferably further comprises:
an optical focusing element to focus the combined radiation from the first radiation source and the radiation from the second radiation source towards the distal portion of the hollow waveguide assembly.

11. The radiation system of claim 7, further comprising:
a housing to retain one or more of: the coupling unit and the second radiation source.

12. The radiation system of claim 11, further comprising a purge gas mechanism to perform one or more of: cool the hollow waveguide assembly, clean the hollow waveguide assembly and clean the housing.

13. The radiation system of claim 12, wherein the purge gas mechanism comprises:
a gas entrance port defined in a first location on an exterior of the housing to enable coupling of a first purge gas into the coupling unit to be directed to the distal portion of the hollow waveguide assembly; and
a gas exit port defined in a second location on the exterior of the housing to enable a second gas received in the coupling unit through the proximal portion of the hollow waveguide assembly to be removed from the coupling unit.

14. The radiation system of claim 13, wherein the purge gas mechanism further comprises:
an isolation wall to define a first chamber and a second chamber inside the housing, the first chamber being decoupled from the second chamber such that the first gas delivered into the first chamber is substantially prevented from entering the second chamber, and such that the second gas delivered into the second chamber is substantially prevented from entering the first chamber.

15. The radiation system of claim 7, wherein the distal portion of the hollow waveguide assembly comprises:
a disposable hollow waveguide coupleable to the coupling unit through a coupler.
